# EUROPEAN PATENT SPECIFICATION

(11) **EP 3 187 487 B1**
(45) Date of publication and mention of the grant of the patent: **22.04.2020**
(21) Application number: 15836787.0
(22) Date of filing: 28.08.2015
(51) Int. Cl.: C07D 233/58, C07C 309/80, C07C 217/08, C07D 295/037, C07C 211/63, H01M 10/052, H01M 10/056, H01M 4/13

(54) **IONIC LIQUID AND PLASTIC CRYSTAL**
IONISCHE FLÜSSIGKEIT UND PLASTISCHER KRISTALL
LIQUIDE IONIQUE ET CRISTAL PLASTIQUE

(30) Priority: 29.08.2014 JP 2014176301
(43) Date of publication of application: 05.07.2017
(73) Proprietor: National Institute of Advanced Industrial Science and Technology, Tokyo 100-8921 (JP)
(72) Inventor: MATSUMOTO, Hajime, Ikeda-shi Osaka 563-8577 (JP); MIZUKADO, Junji, Tsukuba-shi Ibaraki 305-8561 (JP); WANG, Peng-cheng, Tsukuba-shi Ibaraki 305-8561 (JP)
(74) Representative: Winter, Brandl, Fürniss, Hübner, Röss, Kaiser, Polte - Partnerschaft mbB
(86) International application number: PCT/JP2015/074419
(87) International publication number: WO 2016/031961

(56) References cited:
- WO-A1-2009/136608
- JP-A- H10 279 554
- JP-A- 2008 071 546
- JP-A- 2008 277 001
- US-A1- 2010 099 031
- DATABASE CAPLUS [Online] CHEMICAL ABSTRACTS SERVICE, COLUMBUS, OHIO, US; WATANABE, TAKESHI: "Ion conducting polymer electrolyte containing room-temperature molten salt and secondary lithium battery", XP002778904, retrieved from STN Database accession no. 2008:375761 & JP 2008 071546 A (SUMITOMO BAKELITE CO) 27 March 2008 (2008-03-27)
- HAJIME MATSUMOTO ET AL.: 'Fluorosulfonyl-ki o Fukumu Shinki Perfluoro Anion kara naru Ion Ekitai' 2014 NEN ABSTRACTS OF AUTUMN MEETING OF THE ELECTROCHEMICAL SOCIETY OF JAPAN 27 September 2014, page 62, XP009500614
- HAJIME MATSUMOTO ET AL.: 'Trifluorosulfonylmethide kara naru Onium-en no Denkaieki Tokusei' DAI 55 KAI ABSTRACTS, BATTERY SYMPOSIUM IN JAPAN 19 November 2014, page 371, XP009500609

## Description

### Technical Field

The present invention relates to a plastic crystal. The present invention further relates to a non-aqueous electrolyte for lithium secondary batteries and to a lithium secondary battery.

### Background Art

It is important to use a lithium secondary battery at a low temperature, and the use of an ionic liquid as an electrolyte has been proposed (Patent Literature 1). Meanwhile, photovoltaics, cogeneration systems, and the like have become prevalent, and the need has arisen for electric power generation systems that are usable at times of disaster such as the Great East Japan Earthquake; thus, stationary lithium secondary batteries, including those for automotive use, have been increasingly in demand. Lithium secondary batteries that use an organic solvent have already been put into practical use, but they generate heat when charged and discharged, which requires safety measures to prevent the batteries from having a high temperature. In particular, there has recently been an increasing need for high-capacity stationary electrical storage devices, and it is important to prepare a means for making the installation area as small as possible. To achieve an operating temperature of 45°C or lower in terms of a battery that uses an organic solvent, it is necessary to provide a pack battery that is capable of controlling heat and to forcibly air-cool it with a fan. These arrangements, however, cause problems when such batteries are combined in a large-scale system, no matter how large the capacity density of the developed pack battery. Specifically, overall capacity would be reduced, and electrical power for cooling lithium secondary batteries would be consumed. Cooling would not be required if it is possible to develop an electrolyte that is stably operated within a temperature range of 85°C or higher.

An ionic liquid that contains no solvent molecule, has thermal resistance without being vaporized by heating and achieves higher conductivity when the temperature increases. Given this, such an ionic liquid is gathering attention as an electrolyte solution that enables stable operation at high temperatures (Patent Literature 2). Although a solid battery with a solid electrolyte has currently been developed as an excellently safe battery free from liquid leakage, previously known solid electrolytes are of inorganic materials, which are problematically susceptible to impact and likely to crack. From among salts that have a structure similar to that of an ionic liquid, solids that are in a plastic crystal phase near ordinary temperature have become known, and those obtained by adding a lithium salt to these solids are gaining attention as a flexible solid electrolyte with relatively high conductivity (Patent Literature 3). However, to construct a salt that is in the plastic crystal phase within a wide temperature range from a temperature as low as minus several tens degrees to about 150°C, which is the temperature range in which batteries are expected to be operated, ions, in particular anions, that easily achieve molecular motion within the crystal, are necessary.

Although it can be operated at low temperature, an organic solvent electrolyte, which currently serves as the electrolyte of a lithium secondary battery, has a problem in terms of stability at high temperature since a volatile, inflammable organic solvent is used as the electrolyte solution.

Patent Literature 4 discloses an ionic liquid that contains a phosphonium ion having a P-N bond as a cation component.

Patent Literature 5 discloses an ionic liquid that contains an oxazole ring as a cation component.

Patent Literature 6 discloses an ionic liquid that contains sulfonium, ammonium, or phosphonium as a cation component.

Patent Literature 7 discloses an ionic liquid that contains uronium or thiouronium cations as a cation component.

Patent Literature 8 discloses ionic conductive electrolytes for secondary lithium batteries using a [Py₁₃]⁺ salt with [C(SO₂F)₃]⁻ which is molten at room temperature.

Patent Literature 9 discloses anions such as [C(SO₂F)₃]⁻ and cations such as pyrrolidinium, piperidinium, ammonium and imidazolium for use in non-aqueous electrolytes for secondary batteries.

### Citation List

### Patent Literature

PTL 1: JP 2981545 B
PTL 2: JP 2013-196922 A
PTL 3: WO 2008/081811 (Japanese Patent No. 4997610)
PTL 4: JP 5265197 B
PTL 5: JP 2008-130623 A
PTL 6: JP 2008-277001 A
PTL 7: JP 4723480 B
PTL 8: JP 2008-071546 A
PTL 9: US 2010/099031 A1

### Summary of Invention

### Technical Problem

An object of the present invention is to provide a lithium secondary battery, and a non-aqueous electrolyte for lithium secondary batteries, that are stably operable within an intermediate-temperature or high-temperature range (i.e., 85°C or higher), which is extremely difficult to achieve with a conventional organic electrolyte solution.

Another object of the present invention is to provide a plastic crystal.

### Solution to Problem

The present inventors found that a salt that comprises [C(SO₂F)₃]⁻ (hereinbelow this anion is sometimes abbreviated as "[f₃C]⁻") as an anion, and at least one member selected from the group consisting of 1-ethyl-3-methylimidazolium ([EMI]⁺), N-methyl-N-propylpiperidinium ([PP₁₃]⁺), tetramethylammonium ( [N₁₁₁₁]⁺) , tetraethylammonium ([N₂₂₂₂]⁺) and N-methyl-N-ethylpyrrolidinium ([Py₁₂]⁺) as a cation serves as an ionic liquid or plastic crystal. The inventors also found that when used as a non-aqueous electrolyte for lithium secondary batteries, the ionic liquid or plastic crystal achieves high stability and high durability, and even when used for a long period of time, achieves a sufficiently low rate of increase in the interfacial charge transfer resistance of the battery caused by degradation. A plastic crystal is a kind of mesophase in a state in which rotational motion of the molecules or ions freely occur in the solid while translational motion is frozen. Plastic crystals are recently gathering attention as novel solid electrolytes because ions constituting a plastic crystal and metal cations doped to the plastic crystal can travel relatively easily via lattice defects present in the solid.

The present invention provides the following plastic crystal, non-aqueous electrolyte for lithium secondary batteries, and lithium secondary battery.
Item 1. A lithium secondary battery comprising a Li metal negative electrode as a negative electrode and a non-aqueous electrolyte for lithium secondary batteries, the electrolyte comprising an anion and a cation, the anion comprising [C(SO₂F)₃]⁻, and the cation comprising 1-ethyl-3-methylimidazolium ([EMI]⁺).
Item 2. A lithium secondary battery according to Item 1, wherein the non-aqueous electrolyte for lithium secondary batteries further comprises Li[C(SO₂F)₃].
Item 3. A plastic crystal comprising an anion and a cation, the anion comprising [C(S0₂F)₃₎]⁻, and the cation comprising at least one member selected from the group consisting of N-methyl-N-propylpiperidinium ([PP₁₃]⁺), tetramethylammonium ([N₁₁₁₁]⁺) , tetraethylammonium ([N₂₂₂₂]⁺) and N-methyl-N-ethylpyrrolidinium ([Py₁₂]⁺) .
Item 4. A non-aqueous electrolyte for lithium secondary batteries, the electrolyte comprising an anion and a cation, the anion comprising [C(SO₂F)₃]⁻, and the cation comprising at least one member selected from the group consisting of N-methyl-N-propylpiperidinium ([PP₁₃]⁺), tetramethylammonium ([N₁₁₁₁] ⁺) , tetraethylammonium ([N₂₂₂₂]⁺) and N-methyl-N-ethylpyrrolidinium ([Py₁₂]⁺) .
Item 5. The non-aqueous electrolyte for lithium secondary batteries according to Item 4, further comprising Li [C (SO₂F)₃].
Item 6. A lithium secondary battery comprising the non-aqueous electrolyte of any one of Items 4 and 5.
Item 7. The lithium secondary battery according to Item 6, comprising a Li metal negative electrode as a negative electrode.

### Advantageous Effects of Invention

The ionic liquid or plastic crystal of the present invention is capable of being stably operated within an intermediate temperature range as an electrolyte for lithium secondary batteries; thus, it is possible to suppress the need for cooling inside a lithium secondary battery case, making it possible to provide a lithium secondary battery with sufficient storage efficiency per unit volume. The ionic liquid or plastic crystal of the present invention is particularly excellent when used, in particular, for high-capacity lithium secondary batteries such as those for automotive applications, since operation within an intermediate-temperature to high-temperature range is possible, a reduction in the viscosity of the electrolyte at high temperature is possible, and closely assembling lithium secondary batteries is possible to achieve high efficiency.

### Brief Description of Drawings

Fig. 1: Cyclic voltammogram of Li[f₃C]-containing [EMI] [f₃C] on a platinum electrode.
Fig. 2: Cyclic voltammogram of Li[f₃C] -containing [EMI] [f₃C] on a nickel electrode.
Fig. 3: Electrochemical AC impedance spectra of Li metal symmetrical cells.
Fig. 4: 1C Charge-discharge cyclic performance of LiCoO₂/Li cells, in terms of [EMI] [f₃C] and [EMI] [Tf₂N] .
   Upper graph: Cycle dependency of discharge capacity.
   Lower graph: Charging and discharging coulomb efficiency.
Fig. 5: AC impedance plots of charged LiCoO₂/Li cells.
   a) [EMI] [f₃C]
   b) [EMI] [Tf₂N]
Fig. 6: Thermogravimetric changes in a nitrogen atmosphere.
Fig. 7: DSC measurement results of [EMI] [f₃C] and [EMI] [Tf₃C] .
Fig. 8: DSC measurement results of [DEME] [f₃C] and [DEME] [Tf₃C] .
Fig. 9: DSC measurement results of [Py₁₃][f₃C], [PP₁₃][f₃C], [Py₁₃][Tf₃C], and [PP₁₃][Tf₃C] .

### Description of Embodiments

The anion of the ionic liquid or plastic crystal of the present invention comprises [f₃C]⁻, and the cation comprises at least one member selected from [EMI]⁺, [PP₁₃]⁺, [N₁₁₁₁₁]⁺, [N_{2222]}⁺ and [Py₁₂]⁺. The ionic liquid is obtained when the cation is [EMI]⁺, , and the plastic crystal is obtained when the cation is any of [PP₁₃]⁺, [N₁₁₁₁]⁺, [N₂₂₂₂]⁺, and [P_{Y12}]⁺.

The anion may consist only of [f₃C]⁻, or a combination of [f₃C]⁻ and other anions. Examples of other anions include FSI-([(FSO₂)₂N]⁻), TFSI⁻ ([(CF₃SO₂) ₂N]⁻), FTA⁻ ([ (FSO₂) (CF₃SO₂)N]⁻) and Tf₃C⁻ ([(CF₃SO₂)₃C]⁻) . The content of the [f₃C] anion is preferably 50 mol% or more, preferably 70 mol% or more, more preferably 80 mol% or more, even more preferably 90 mol% or more, particularly preferably 95 mol% or more, and most preferably 100 mol%, of the total anion.

The cation is more preferably [EMI]⁺, and [PP_{13]}⁺, and still more preferably [EMI]⁺. It is also possible to combine [EMI]⁺ with at least one member selected from [DEME]⁺, [Py₁₃]⁺, [PP₁₃]⁺, [N₁₁₁₁]⁺, [N₂₂₂₂]⁺, [N₆₁₁₁]⁺, [N₆₂₂₂]⁺, [Py₁₂]⁺, [C₄mim]⁺, and [C₆mim]⁺. In one preferable embodiment, the content of [EMI]⁺ in the total cation is 30 mol% or more, preferably 50 mol% or more, more preferably 70 mol% or more, still more preferably 90 mol% or more, particularly preferably 95 mol% or more, and most preferably 100 mol%.

The following are the abbreviations and structural formulas of cations and anions used in the present specification.

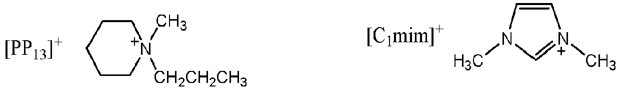

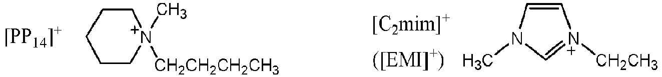

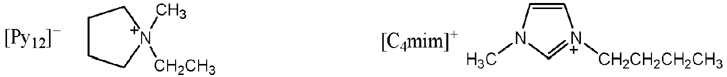

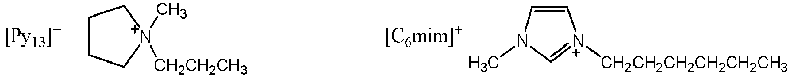

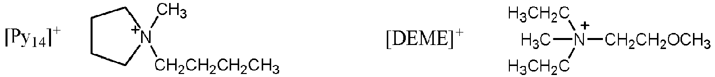

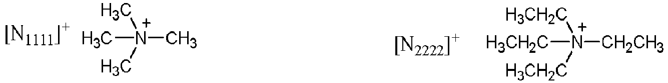

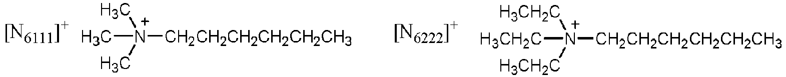

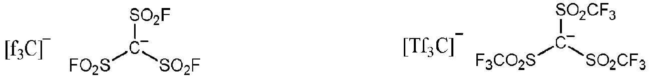

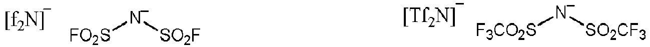

In this specification, an ionic liquid refers to a substance that is liquid at room temperature (melting point: 35°C or lower), and a plastic crystal undergoes, at a temperature lower than the above melting point, a solid-solid phase transition accompanied by a great calorimetric change that is equivalent to a calorific value obtained when a general solid or liquid is subjected to melting, and achieves, as a result, a melting entropy of about 20 J K-¹mol⁻¹ or less. To be identified as the plastic crystal of the present invention, one or both of the following requirements must be satisfied, i.e., undergoing solid-solid phase transition at a temperature lower than the melting point, and achieving a melting entropy of about 20 J K⁻¹ mol⁻¹ or less (J. Timmermans, J. Phys. and Chem. Solids, 18(1), (1961)). A plastic crystal, which is completely different from general crystalline materials, is characterized as being a flexible, sticky solid.

More specifically, the ionic liquid is [EMI][f₃C], and the plastic crystal is [PP₁₃][f₃C], [N₁₁₁₁][f₃C], [N₂₂₂₂][f₃C], and [Py₁₂][f₃C].

An alkali metal salt comprising [f₃C]⁻ is a known substance and may be produced by a known production method. The following is a preferable production scheme.

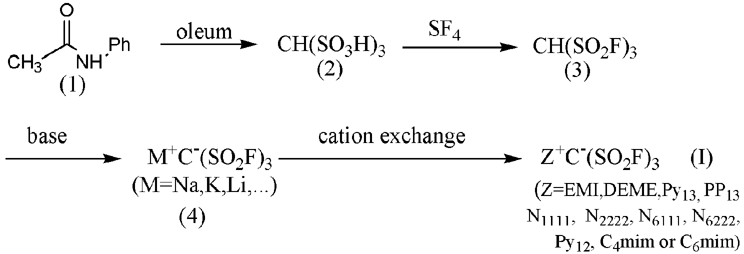

In the formula, M represents an alkali metal, and preferably Na, K, or Li; Z represents [EMI]⁺, [DEME]⁺, [Py₁₃]⁺, [PP₁₃]⁺, [N₁₁₁₁]⁺, [N₂₂₂₂]⁺, [N₆₁₁₁]⁺, [N₆₂₂₂]⁺, [PY₁₂]⁺, [C₄mim]⁺, or [C₆mim]⁺.

Compound (1), which is a starting material, is sulfated with oleum to yield Compound (2), which is reacted with SF₄ to obtain Compound (3). Compound (3) is reacted with a base such as alkali metal carbonate or alkali metal hydrogen carbonate (e.g., sodium carbonate, sodium hydrogen carbonate, potassium carbonate, potassium hydrogen carbonate, lithium carbonate, or lithium hydrogen carbonate) to form a salt (4) of an alkali metal (M), which is then reacted with [EMI]⁺, [DEME]⁺, [Py₁₃]⁺, [PP₁₃]⁺, [N₁₁₁₁]⁺, [N₂₂₂₂]⁺, [N₆₁₁₁]⁺, [N₆₂₂₂]⁺, [PY₁₂]⁺, [C₄mim]⁺, or [C₆mim]⁺ to perform cation exchange. In this manner, a target ionic liquid or plastic crystal of the present invention is obtained.

The lithium secondary battery of the present invention has active material layers on collectors, and the ionic liquid or plastic crystal of the present invention is used as the electrolyte. One electrode is separated by a separator.

As a collector usable as a positive electrode, it is possible to use metal sheet, such as aluminum, stainless steel, nickel, and titanium. In addition, it is preferable to use aluminum and stainless steel whose surface is coated with carbon, nickel, titanium, or silver, and alloys obtained by incorporating carbon, nickel, titanium, or silver into the surface of the aluminum or stainless steel.

As a collector used as a negative electrode, it is preferable to use copper, stainless steel, nickel, and titanium. The collector is usually used in the form of a film or a sheet; however, a porous body or a foam may also be used. The thickness of the collector is not particularly limited, and is preferably 1 to 500 µm. The surface of the collector is preferably provided with irregularities by a surface treatment.

Examples of positive electrode active materials include Li₀.₃MnO₂, Li₄Mn₅O₁₂, V₂O₅, LiCoO₂, LiMn₂O₄, LiNiO₂, LiFePO₄, LiCO_{1/3}Ni_{1/3}Mn_{1/3}O₂, Li_{1.2}(Fe_{0.5}Mn_{0.5})_{0.8}O₂, Li_{1.2}(Fe_{0.4}Mn_{0.4}Ti₀.₂)_{0.8}O₂, Li₁₊ₓ(Ni_{0.5}Mn_{0.5})₁₋ₓO₂, LiNi_{0.5}Mn_{1.5}O₄, Li₂MnO₃, Li_{0.76}Mn_{0.5}1Ti_{0.49}O₂, LiNi_{0.8}CO_{0.15}Al_{0.05}O₂, Fe₂O₃, LiCoPO₄, LiMnPO₄, Li₂MPO₄F (M = Fe, Mn), LiMn_{0.875}Fe_{0.125}PO₄, Li₂FeSiO₄, Li₂₋ₓMSi₁₋ₓPₓO₄(M = Fe, Mn), LiMBO₃(M = Fe, Mn), FeF₃, Li₃FeF₆, Li₂TiF₆, Li₂FeS₂, TiS₂, MOS₂, and FeS (however, x is within the range of 0 to 1).

The negative electrode active material is not particularly limited, and known negative electrode active materials may be used. Examples of negative electrode active materials preferably used in the non-aqueous electrolyte lithium secondary battery of the present invention include carbon materials, as well as metal oxides and metal nitrides that are capable of incorporating lithium ions. Examples of carbon materials include natural graphite, artificial graphite, pyrolytic carbons, cokes, meso-carbon microbeads, carbon fibers, active carbons, and pitch-coated graphite. Examples of metal oxides capable of incorporating lithium ions include metal compounds that contain tin or silicon, such as tin oxide and silicon oxide. Examples of metal nitrides include Li_{2.6}CO_{0.4}N. Examples also include a mixture comprising graphite, a tin alloy, and a binding agent; a silicon thin film; and a lithium foil.

The active material layers contain the positive electrode active material or negative electrode active material described above, and preferably further contain a conducting agent and a binding agent. A filler and a lithium salt may also be incorporated as additional materials. Examples of a conducting agent include natural graphite, artificial graphite, carbon black, acetylene black, ketjen black, carbon fibers, metal powders, metal fibers, and polyphenylene derivatives. Examples of a binding agent include water-soluble polymers such as carboxymethyl cellulose, cellulose, diacetyl cellulose, methyl cellulose, hydroxyethyl cellulose, hydroxypropyl cellulose, sodium alginate, polyacrylic acid, sodium polyacrylate, polyvinyl phenol, polyvinyl methyl ether, polyvinyl alcohol, polyvinyl pyrrolidone, polyacrylonitrile, polyacrylamide, polyhydroxy(meth)acrylate, and styrene-maleic acid copolymer; emulsions (latexes), such as polyvinyl chloride, polytetrafluoroethylene, polyvinylidene fluoride (PVDF), tetrafluoroethylene-hexafluoropropylene copolymer, vinylidene fluoride-tetrafluoroethylene-hexafluoropropylene copolymer, polyethylene, polypropylene, ethylene-propylene-diene terpolymer (EPDM), sulfonated EPDM, polyvinyl acetal resin, (meth)acrylic acid ester-containing (meth)acrylic acid ester copolymers, such as methyl methacrylate and 2-ethylhexyl acrylate, vinyl ester-containing polyvinyl ester copolymers, such as (meth)acrylic acid ester-acrylonitrile copolymer and vinyl acetate, styrene-butadiene copolymers, acrylonitrile-butadiene copolymers, polybutadiene, neoprene rubber, fluororubber, polyethylene oxide, polyester-polyurethane resin, polyether-polyurethane resin, polycarbonate-polyurethane resin, polyester resin, phenol resin, and epoxy resin.

The filler is not particularly limited, as long as it is a fibrous material that does not undergo a chemical change in the non-aqueous electrolyte secondary battery of the present invention. Usually, olefin-based polymers, such as polypropylene and polyethylene, and fibers, such as glass and carbon, are used. The amount of the filler added is not particularly limited, and is preferably 0 to 30 mass%.

Examples of lithium salts include those that exhibit thermal resistance at a temperature within an intermediate temperature range, such as LiBF₄, LiCF₃SO₃, LiC (CF₃SO₂)₃, LiC(FSO₂)₃, LiN(CF₃SO₂)₂, LiN(FSO₂)₂, LiN(CF₃CF₂SO₂)₂, LiN(CF₃SO₂)(C₂F₅SO₂), LiN(CF₃SO₂)(C₄F₉SO₂), LiN(CF₃CF₂CO)₂, and LiBOB. These salts may be used alone, or in a combination of two or more. A preferable lithium salt is LiC(FSO₂)₃.

In a preferable embodiment, the lithium secondary battery of the present invention may have a structure of a laminate cell itself. It is preferable that the separator has excellent capability of being impregnated with an ionic liquid, and has excellent thermal resistance. Specific examples of the separator include a silica nanoparticle-containing polyolefin separator and an inorganic glass filter.

Preferable examples of the positive electrode active material of a battery include LiFePO₄, which has excellent thermal resistance.

It is assumed that batteries that are operable at room temperature may be operable as is at high temperatures. In fact, however, batteries that use a previously known organic solvent electrolyte solution cannot be operated at high temperatures, and must be sufficiently air-cooled. Further, the formation of an air pathway reduces the space filling rate; thus, even if it is possible to achieve a great capacity per unit cell, the efficiency of the whole system is reduced. The non-aqueous electrolyte for lithium secondary batteries of the present invention, which has excellent thermal resistance and excellent durability, contributes to the development of a battery with excellent thermal resistance, and is preferable, in particular, for use in large-scale stationary storage batteries because the efficiency greatly improves.

The lithium secondary battery of the present invention is applicable to any shape, such as a sheet, a square, and a cylinder. The shape of the electrode may also be optimally selected according to the shape of the non-aqueous electrolyte secondary battery.

The positive electrode active material layer and the negative electrode active material layer are provided on a collector. The positive electrode active material layer and negative electrode active material layer may be provided on one surface or both surfaces of a collector. It is more preferable to use an electrode having positive and negative electrode active material layers on both surfaces. There is no particular limitation to the size of the negative-electrode plate relative to the size of the positive-electrode plate. The positive electrode plate area is preferably 0.9 to 1.1, and particularly preferably 0.95 to 1.0, based on the positive electrode plate area being 1. The electrode is obtained by applying an active material-containing coating liquid to the surface of a collector, followed by drying and further pressing to form an active material layer.

As the coating liquid, for example, a slurry coating liquid may be used, optionally comprising a conducting agent mentioned above, a binding agent mentioned above, and a dispersion medium, such as N-methyl-2-pyrrolidone (NMP), water, and toluene.

Examples of the application method include reverse roll coating, direct roll coating, blade coating, knife coating, extrusion coating, curtain coating, gravure coating, bar coating, dip coating, and squeeze coating. Of these, blade coating, knife coating, and extrusion coating are preferable. The application is preferably performed at a rate of 0.1 to 100 m/min. The application method may be selected from the above in view of the solution properties and drying properties of the coating liquid, and in this way, it is possible to obtain an excellent surface state of the coating layer. The application of the coating liquid may be performed sequentially with respect to one surface at a time or both surfaces simultaneously.

The electrolyte solution of a lithium secondary battery may be obtained, for example, by dissolving or mixing an alkali metal salt (supporting electrolyte) that is believed to exhibit desired thermal resistance with an ionic liquid or plastic crystal that contains an anion, such as [f₃C]⁻, which shows excellent thermal resistance. These can be uniformly mixed by heating to the melting point or higher of a mixed salt that is obtained by mixing with a salt given as an example in the present invention. Examples of anions of the supporting electrolyte include [BF₄]⁻, [(FSO₂)₂N]⁻, [(FSO₂)(CF₃SO₂)N]⁻, [(CF₃SO₂)₂N]⁻, [(CF₃CF₂SO₂)₂]N⁻, [(C₂H₄O₂)₂B]⁻, [(CF₃SO₂)₃C]⁻, and [CF₃SO₃]⁻. Examples further include [f₃C]⁻, i.e., the anion of the electrolyte of the present invention. As the anion of the supporting electrolyte, it is preferable to use [f₃C]⁻, which is the same anion used in the electrolyte of the present invention. As the cation of the supporting electrolyte, it is preferable to use Li⁺.

In a preferable embodiment, the lithium secondary battery of the present invention has a laminate structure in which the positive electrode active material layer described above is provided on one surface of a separator, and the negative electrode active material layer is provided on the other surface of the separator. Further, a collector is provided on the active material layer surfaces opposite to the separator. The separator layer is impregnated with an electrolyte mixture that comprises a supporting electrolyte and the electrolyte of the present invention, the electrolyte mixture being in a dissolution state by heating if necessary.

The laminate structure is not limited to a simple, single layer laminate, and may be, for example, a multilayer laminate structure that comprises a plurality of these laminate structures, a structure that comprises a combination of laminates in which layers are formed on both surfaces of a collector, and a structure obtained by winding these formations.

A non-portable (stationary) lithium secondary battery has a multilayer laminate structure. However, the electrolyte of the present invention, which is highly stable at a temperature within an intermediate temperature range, can reduce or eliminate the number of fans for cooling or air pathways for cooling, making it possible to increase the storage capacity per unit volume in a state of an assembled battery in which single cells are assembled.

### Examples

The present invention is described below in more detail with reference to Examples and Comparative Examples.

In Examples and Comparative Examples, commercially available products were used for cation components, i.e., [EMI]⁺, ([C₂mim]⁺), [DEME]⁺, [Py₁₂]⁺, [Py₁₃]⁺, [Py₁₄]⁺, [PP₁₃]⁺, [PP₁₄]⁺ [N₁₁₁₁]⁺, [N₆₁₁₁]⁺, [N₆₂₂₂]⁺, [Cᵢmim]⁺, [C₄mim]⁺, and [C₆mim]⁺; and for anion components, i.e., [Tf₂N]⁻(= [(CF₃SO₂)₂N]⁻), [f₂N]⁻(= [(FSO₂)₂N]⁻), and [Tf₃C]⁻(=[(CF₃SO₂)₃C]⁻).

The thermal gravimetric analysis (TGA) was performed using a Seiko Instruments TG/DTA 6200 in a nitrogen stream at a scan rate of 10°C per minute.

The differential scanning calorimetry (DSC) was performed using a Perkin Elmer Pyris 1 at a scan rate of 10°C per minute.

The thermal resistance was evaluated based on the thermal-decomposition temperature measured by TGA. The state of a liquid, a solid, or a plastic crystal was determined in view of the presence or absence of phase transition temperature, its temperature, and calorimetric change, as measured by DSC.

### Production Example 1: Synthesis of Anion (f₃C)

Compound (2) (58.4 mg), which is a commercially available product obtained from Alcatraz Chemicals (Gujarat, India), was reacted with 194 mg of SF₄ to obtain 51.6 mg of compound (3) . Then, 92.4 mg of compound (3) was reacted with an excess amount of potassium carbonate to form 93.9 mg of potassium salt (4) comprising f₃C anion.

### Example 1

Potassium salt (4) comprising f₃C anion obtained in Production Example 1 was reacted with an equimolar bromide of [EMI]⁺, [DEME]⁺, [Py₁₃]⁺, or [PP₁₃]⁺ to perform cation exchange. In this manner, a target ionic liquid ([EMI] [f₃C]) of the present invention and target plastic crystals ([DEME] [f₃C] (reference), [Py₁₃][f₃C] (reference), and [PP₁₃][f₃C]) of the present invention were obtained.

The following shows the physical property values of the obtained ionic liquid and plastic crystals. Table 1 shows the melting point, glass transition temperature, and solid-solid phase transition temperature.
1) [EMI][f₃C]
   ¹H-NMR (CD₃CN, 300 MHz): δ = 1.45 (t, J = 7.2 Hz, 3H), 3.81 (s, 3H), 4.16 (q, J = 7.2 Hz, 2H), 7.32 (s, 1H), 7.37 (s, 1H), 8.39 (s, 1H) : ¹⁹F-NMR (CD₃CN, 283 MHz): δ = 71.5 (s, 3F).
   Elemental analysis values (theoretical values): H 2.99% (2.98%); C 22.71% (22.58%); N 7.58% (7.52%); F 15.36% (15.31%). Ionic conductivity at 25°C: 6.2 mS cm⁻¹. Viscosity at 25°C: 39 mPa.s. Density at 25°C: 1.55 g mL⁻¹. Melting entropy: 40 J ⁻¹mol⁻¹. Thermal-decomposition temperature (at the time of 10% reduction): 246°C.
2) [DEME] [f₃C] (reference)
   ¹H-NMR (CD₃CN, 300 MHz): δ = 1.26 (t of t, J = 7.2 Hz and 1.9 Hz, 6H), 2.92 (s, 3H), 3.27-3.37 (complex, 9H), 3.71 (m, 2H) : ¹⁹F-NMR (CD₃CN, 283 MHz) : δ = 71.6 (s, 3F).
   Elemental analysis values (theoretical values): H 4.81% (4.95%); C 26.41% (26.53%); N 3.44% (3.44%); F 14.03% (13.99%). Melting entropy: 6.1 J ⁻¹mol⁻¹. Thermal-decomposition temperature (at the time of 10% reduction): 325°C.
3) [Py_{13]} [f₃C] (reference)
   ¹H-NMR (CD₃CN, 300 MHz): δ = 0.96 (t, J = 7.2 Hz, 3H), 1.75 (m, 2H), 2.15 (m, 4H), 2.93 (s, 3H), 3.17 (m, 2H), 3.39 (m, 4H); ¹⁹F-NMR (CD₃CN, 283 MHz) : δ = 71.5 (s, 3F).
   Elemental analysis values (theoretical values): H 4.63% (4.66%); C 27.67% (27.76%); N 3.47% (3.60%); F 14.88% (14.64%). Melting entropy: 9.3 J K⁻¹mol⁻¹. Thermal-decomposition temperature (at the time of 10% reduction): 343°C.
4) [PP₁₃][f₃C]
   ¹H-NMR (CD₃CN, 300 MHz): δ = 0.963 (t, J = 7.2 Hz, 3H), 1.59-1.88 (m, 8H), 2.92 (s, 3H), 3.14-3.24 (complex, 6H); ¹⁹F-NMR (CD₃CN, 283 MHz) : δ = 71.5 (s, 3F).
   Elemental analysis values (theoretical values): H 4.97% (5.00%); C 29.59% (29.77%); N 3.47% (3.47%); F 14.22% (14.13%). Melting entropy: 8.6 J K⁻¹mol⁻¹. Thermal-decomposition temperature (at the time of 10% reduction): 373°C.

### Example 2

Potassium salt (4) comprising f₃C anion obtained in Production Example 1 was reacted with an equimolar bromide of [N₆₁₁₁]+, [N₆₂₂₂]⁺, [N₁₁₁₁]⁺, [Py₁₂]⁺, [C₄mim]⁺, and [C₆mim]⁺ to perform cation exchange. In this manner, target ionic liquids [N₆₁₁₁][f₃C] (reference), [N₆₂₂₂]⁺[f₃C] (reference), [N₁₁₁₁[f₃C], [N₂₂₂₂][f₃C], [PP₁₄][f₃C] (reference), [Py₁₂][f₃C], [Py₁₄][f₃C] (reference), [C₁mim][f₃C] (reference), [C₄mim][f₃C] (reference), and [C₆mim][f₃C] (reference) were obtained. The following shows the physical property values of the obtained ionic liquids or plastic crystals. Table 1 shows the melting point, glass transition temperature, and solid-solid phase transition temperature.
1) [N₁₁₁₁][f₃C]
   ¹H NMR (DMSO-d6, 300 MHz): δ = 3.06 (s, 12H); ¹⁹F NMR (DMSO-d6, 283 MHz) : δ = 71.9 (s, 3F).
   Thermal-decomposition temperature (at the time of 10% reduction): 348°C.
2) [N₂₂₂₂][f₃C]
   ¹H NMR (CDCl₃, 300 MHz): δ = 1.35 (t, J = 7.2 Hz, 4 × 3H), 3.23 (q, J = 7.2 Hz, 4 × 2H); ¹⁹F NMR (CDCl₃, 283 MHz): δ = 71.2 (s, 3F) .
   Thermal-decomposition temperature (at the time of 10% reduction): 317°C.
3) [N₆₁₁₁][f₃C] (reference)
   ¹H NMR (CDCl₃, 300 MHz): δ = 0.91 (t, J = 7.2 Hz, 3H), 1.15-1.47 (complex, 3 × 2H), 1.76 (m, 2H), 3.13 (s, 3 × 3H), 3.26 (m, 2H); ¹⁹F NMR (CDCl₃, 283 MHz): δ = 71.5 (s, 3F).
   Thermal-decomposition temperature (at the time of 10% reduction): 346°C.
4) [N₆₂₂₂][f₃C] (reference)
   ¹H NMR (CDCl₃, 300 MHz): δ = 0.91 (t, J = 7.2 Hz, 3H), 1.23-1.41 (complex, 3 × 3H, 3 × 2H), 1.64 (m, 2H), 3.21 (m, 2H), 3.26 (q, J = 7.2 Hz, 3 × 2H) ; ¹⁹F NMR (CDCl₃, 283 MHz): δ = 71.2 (s, 3F). Thermal-decomposition temperature (at the time of 10% reduction): 340°C.
5) [Py₁₂][f₃C]
   ¹H NMR (DMSO-d6, 300 MHz): δ = 1.24 (t, J = 7.2 Hz, 3H), 2.04 (br, 2 ×2H), 2.92 (s, 3H), 3.23-3.50 (complex, 3 × 2H); ¹⁹F NMR (DMSO-d6, 283 MHz): δ = 71.9 (s, 3F).
   Thermal-decomposition temperature (at the time of 10% reduction): 284°C.
6) [C₄mim][f₃C] (reference)
   ¹H NMR (DMSO-d6, 300 MHz): δ = 0.87 (t, J = 7.2 Hz, 3H), 1.21 (m, 2H), 1.73 (m, 2H), 3.81 (s, 3H), 4.13 (t, J = 7.2 Hz, 2H), 7.67 (t, J = 1.7 Hz, 1H), 7.73 (t, J = 1.7 Hz, 1H), 9.07 (s, 1H) ; ¹⁹F NMR (DMSO-d6, 283 MHz): δ = 71.8 (s, 3F).
   Thermal-decomposition temperature (at the time of 10% reduction): 320°C.
7) [C₆mim][f₃C] (reference)
   ¹H NMR (DMSO-d6, 300 MHz): δ = 0.83 (t, J = 7.2 Hz, 3H), 1.15-1.32 (complex, 3 × 2H), 1.76 (m, 2H), 3.81 (s, 3H), 4.12 (t, J = 7.2 Hz, 2H), 7.66 (t, J = 1.7 Hz, 1H), 7.73 (t, J = 1.7 Hz, 1H), 9.07 (s, 1H) ; ¹⁹F NMR (DMSO-d6, 283 MHz): δ = 71.9 (s, 3F). Thermal-decomposition temperature (at the time of 10% reduction): 327°C.

### Comparative Example 1

Potassium salt (4) comprising f₃C anion obtained in Production Example 1 was reacted with an equimolar bromide of [PP₁₄]⁺, [Py₁₄]¹, and [C₁mim]⁺ to perform salt exchange. In this manner, [PP₁₄][f₃C], [Py₁₄][f₃C], and [C₁mim][f₃C] were obtained. The following shows the physical property values of the obtained ionic liquids or plastic crystals. Table 1 shows the melting point, glass transition temperature, and solid-solid phase transition temperature.
1) [PP₁₄][f₃C]
   ¹H NMR (DMSO-d6, 300 MHz): δ = 0.91 (t, J = 7.2 Hz, 3H), 1.29 (m, 2H), 1.49 (m, 2H), 1.61 (m, 2H), 1.74 (br, 2 × 2H), 2.94 (s, 3H), 3.16-3.42 (br, 3 × 2H); ¹⁹F NMR (DMSO-d6, 283 MHz): δ = 71.9 (s, 3F) .
   Thermal-decomposition temperature (at the time of 10% reduction): 349°C.
2) [Py₁₄][f₃C]
   ¹H NMR (DMSO-d6, 300 MHz): δ = 0.90 (t, J = 7.2 Hz, 3H), 1.27 (m, 2H), 1.64 (m, 2H), 2.05 (br, 2 ×2H), 2.94 (s, 3H), 3.30 (t, J = 7.2 Hz, 2H), 3.40 (m, 2 × 2H); ¹⁹F NMR (DMSO-d6, 283 MHz): δ = 71.9 (s, 3F).
   Thermal-decomposition temperature (at the time of 10% reduction): 351°C.
3) [C₁mim][f₃C]
   ¹H NMR (DMSO-d6, 300 MHz): δ = 3.81 (s, 2 × 3H), 7.64 (s, 1H), 7.65 (s, 1H), 8.99 (s, 1H); ¹⁹F NMR (DMSO-d6, 283 MHz): δ = 71.9 (s, 3F).
   Thermal-decomposition temperature (at the time of 10% reduction): 333°C.

### Comparative Example 2

A commercially available potassium salt comprising Tf₃C anion was reacted with an equimolar bromide of [EMI]⁺, [N₁₁₁₁]⁺, [N₆₁₁₁]⁺, [N₆₂₂₂]⁺, [PP₁₄]⁺, [P_{Y12}]⁺, [Py₁₄]⁺, [C₁mim]⁺, [C₄mim]⁺, and [C₆mim]⁺ to perform salt exchange. In this manner, [EMI] [Tf₃C], [N₁₁₁₁][Tf₃C], [N₆₁₁₁][Tf₃C], [N₆₂₂₂][Tf₃C], [PP₁₄][Tf₃C], [Py₁₂][Tf₃C], [Py₁₄][Tf₃C], [C₁mim][Tf₃C], [C₄mim][Tf₃C], and [C₆mim][Tf₃C] were obtained. Table 2 shows the melting point and Tg of the obtained Tf₃C salts.

### Comparative Example 3

A commercially available potassium salt comprising f₂N anion was reacted with an equimolar bromide of EMI⁺ to perform cation exchange. In this manner, [EMI][f₂N] was obtained. The stability with respect to the Li metal was evaluated in Test Example 1(2).

### Comparative Example 4

A commercially available potassium salt comprising Tf₂N anion was reacted with an equimolar bromide of EMI⁺ to perform salt exchange. In this manner, [EMI][Tf₂N] was obtained. An evaluation was performed in Test Example 1(2) and 1(3).

**Table 1**

| Phase transition temperature and the state at 35°C | | | | |
|---|---|---|---|---|
| | **f₃C⁻** | | | |
| | State | **Tₘ¹⁾(°C)** | **T_{g}²⁾(°C)** | **Tₛ₋ₛ³⁾(°C)** |
| **C₁mim⁺** | S⁴⁾ | 61 | none⁵⁾ | none |
| **C₂mim (EMI⁺)** | L⁶⁾ | 22 | none | none |
| **C₄mim⁺** | L | 33 | none | none |
| **C₆mim⁺** | L | none | -85 | none |
| **Py₁₂⁺** | PC⁷⁾ | decomp⁸⁾ | none | -63 |
| **Py₁₃⁺** | PC | 177 | none | 24 |
| **Py₁₄⁺** | S | 85 | none | none |
| **PP₁₃⁺** | PC | 146 | none | 9.1 |
| **PP₁₄⁺** | S | 36 | -78 | none |
| **N₁₁₁₁⁺** | PC | decomp | none | -23 |
| **N₂₂₂₂⁺** | PC | decomp | none | -1 |
| **DEME⁺** | PC | 69 | none | -29 |
| **N₆₁₁₁⁺** | L | 29 | None | none |
| **N₆₂₂₂⁺** | L | none | -85 | none |

| | | | | |
|---|---|---|---|---|
| 1) Melting point; 2) glass transition temperature; 3) solid-solid phase transition temperature (only of those that are significantly exothermic (equal to or more than several kJ/mol, which is achieved by a general solid); 4) solid; 5) not observed; 6) liquid; 7) plastic crystal; 8) underwent thermal decomposition before melting. | | | | |

**Table 2**

| Phase transition temperature and the state at 35°C | | | | |
|---|---|---|---|---|
| | **Tf₃C⁻** | | | |
| | State | **Tₘ¹⁾** | **T_{g}²⁾** | **Tₛ₋ₛ³⁾** |
| **C₁mim⁺** | S⁴⁾ | 41 | none⁵⁾ | none |
| **C₂mim (EMI⁺)** | L⁶⁾ | 29 | -72 | none |
| **C₄mim⁺** | L | none | -70 | none |
| **C₆mim⁺** | L | none | -79 | none |
| **Py₁₂⁺** | S | 81 | None | none |
| **Py₁₃⁺** | S | 71 | -59 | none |
| **Py₁₄⁺** | S | 56 | None | none |
| **PP₁₃⁺** | S | 54 | -51 | none |
| **PP₁₄⁺** | S | 37 | -55 | none |
| **N₁₁₁₁⁺** | S | 137 | None | none |
| **DEME⁺** | L | none | -77 | none |
| **N₆₁₁₁⁺** | S | 53 | -48 | none |
| **N₆₂₂₂⁺** | L | none | -55 | none |

| | | | | |
|---|---|---|---|---|
| 1) Melting point; 2) glass transition temperature; 3) solid-solid phase transition temperature (only of those that are significantly exothermic (equal to or more than several kJ/mol, which is achieved by a general solid); 4) solid; 5) not observed; 6) liquid. | | | | |

In Tables 1 and 2, "PC" represents a plastic crystal, "L" represents a liquid, and "S" represents a solid. Also, "decomp" means that it reached the decomposition temperature before melting was observed.

With reference to Table 1, the obtained products were identified as plastic crystals (PCs) when they satisfied the following two points, i.e., the plastic crystal-specific solid-solid phase transition (Tₛ₋ₛ) accompanied by a considerable amount of exothermic heat to melting and endothermic heat to coagulation at low temperature was observed, and it was sticky at room temperature.

### Test Example 1

### (1) Electrochemical Stability

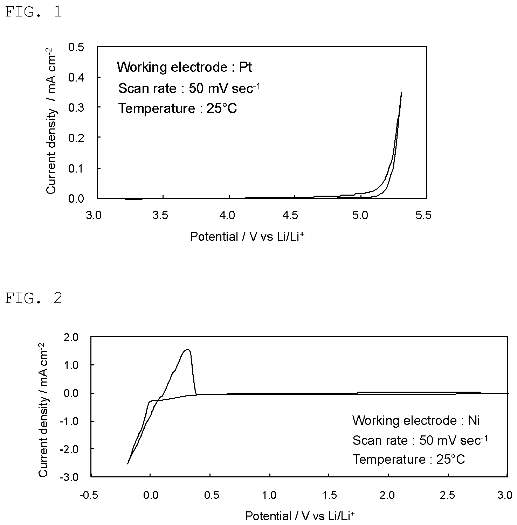

A cyclic voltammogram was measured in [EMI][f₃C] containing 0.37 molkg⁻¹ of Li[f₃C] on a platinum electrode (Fig. 1). Further, a cyclic voltammogram was measured in [EMI][f₃C] containing 0.37 mol kg⁻¹ of Li[f₃C] on a nickel electrode (Fig. 2) .

It was revealed that the coexistence of Li salt allowed the f₃C anion-containing EMI ionic liquids to exhibit high oxidation stability (high enough to be applied to a positive-electrode material with a voltage of about 4.5 V)) and high reduction stability (high enough to enable lithium metal deposition/remelting) .

### (2) Stability with respect to Li Metal

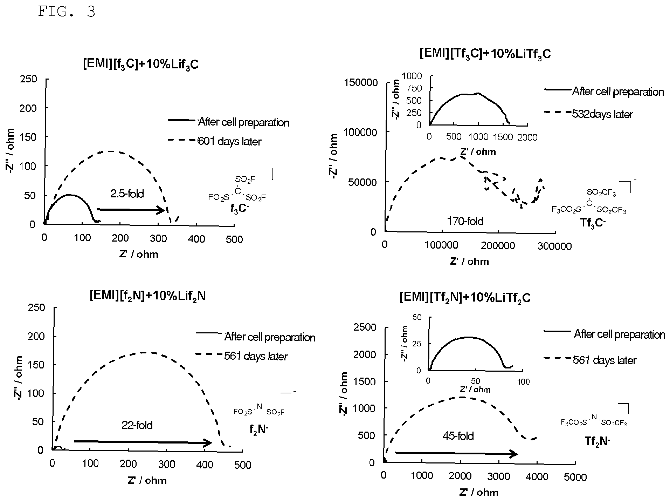

The electrochemical AC impedance spectra (25°C, 500 kHz-0.1 Hz, amplitude: ±10 mV) were measured for Li-metal symmetrical cells containing the [EMI][f₃C] obtained in Example 1, the [EMI][Tf₃C] obtained in Comparative Example 2, the [EMI][f₂N] obtained in Comparative Example 3, or the [EMI][Tf₂N] obtained in Comparative Example 4 (Fig. 3).

The charge transfer reaction rate constant at the Li metal interface is proportional to the reciprocal of the width of a semicircular arc (interfacial charge-transfer resistance) observed in the AC impedance measurement. Thus, an anion that forms a circular arc as small as possible is preferable when Li metal is used as a negative electrode. With reference to this point, a smaller reaction rate on the Li negative electrode is achieved in the following order: a system comprising f₂N⁻, which was 20 Ω after cell preparation (smallest); a system comprising Tf₂N⁻ (80 Ω); a system comprising f₃C⁻ (130 Ω); and a system comprising Tf₃C⁻ (1500 Ω). Li metal is an active metal, forms a solid-electrolyte interface (commonly SEI) through, for example, reduction decomposition caused when in contact with an electrolyte solution, and achieves an effect of inhibiting the decomposition of electrolyte solution. Fig. 3 also shows dotted lines representing the results obtained almost 1.5 year after the preparation of Li metal symmetrical cells, by which a significant increase in the size of circular arcs are confirmed. The system comprising f₃C⁻ of the present invention achieved only a 2.5-fold increase while the other Comparative Examples achieved a 22-fold, 45-fold, or 170-fold increase. This suggests that the solid-electrolyte interface formed by the system comprising f₃C⁻ of the present invention was more excellent than those formed by the other systems, achieving a stable Li metal-ionic liquid interface. Further, in view of the results of Fig. 3, even if the [EMI] [f₂N] was stabile over many cycles, the [EMI] [f₂N] resulted in a high increase in the resistance at the Li electrode side. Considering this, it is assumed that f₃C⁻ is more excellent in terms of long-term storage characteristics of a battery.

### (3) Novel Ionic Liquid LiCoO₂/Li Cycle Test

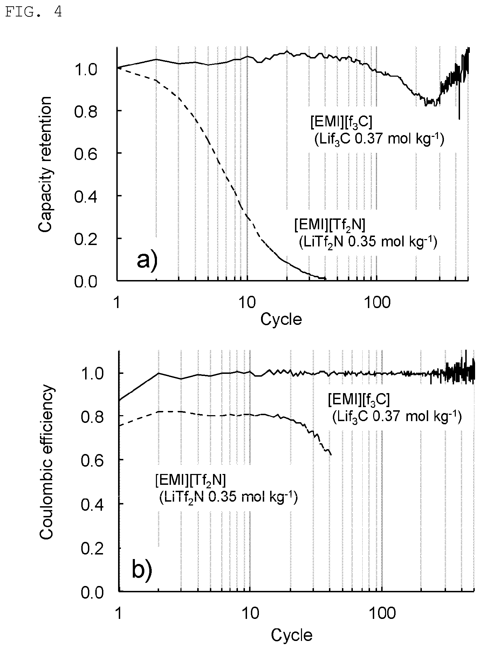

Fig. 4 shows the results of the measurement of 1C charge-discharge cyclic performance of LiCoO₂/Li cells using the [EMI] [f₃C] (Example 1) and the [EMI] [Tf₂N] (Comparative Example 4). Upper graph: cycle dependency of discharge capacity. Lower graph: charging and discharging coulomb efficiency. The [EMI] [Tf₃C] to which Li[Tf₃C] was incorporated showed high interfacial charge-transfer resistance as described in (2), and was thus never active as a battery because of a high internal resistance due to its excessively high viscosity. In contrast, the [EMI] [f₃C] of the present invention was active in an excellent manner and had much more excellent cycle stability than the previously known [EMI] [Tf₂N] . The coulomb efficiency was about 1, which clarifies that the decomposition of the electrolyte solution, deterioration of the electrode were suppressed.

When [EMI] [f₃C] was used, up to 500 cycles were stably achieved with a coulomb efficiency of about 100%. This clarifies the achievement of the stability at the Li interface as shown in Fig. 3, as well as oxidation stability high enough to be applied to a 4 V class positive electrode.

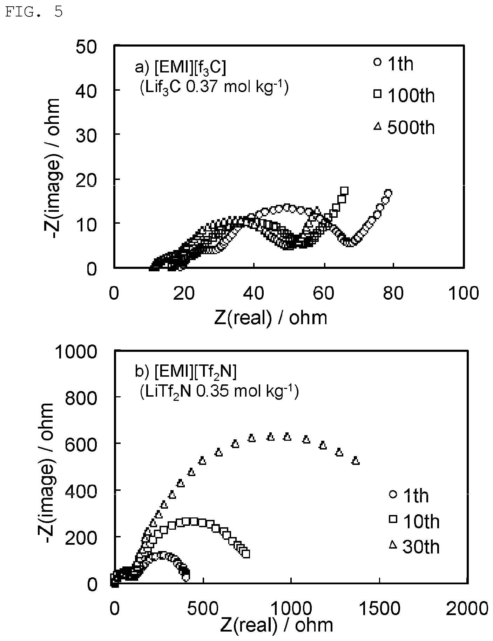

Further, the AC impedance plots in terms of charged LiCoO₂/Li cells were assessed using the [EMI][f₃C] (Example 1), and the [EMI][Tf₂N] (Comparative Example 4) . Fig. 5 shows the results.

The electrode interfacial charge-transfer resistance is determined based on the width of the circular arc at the time of charge; the width of [EMI][f₃C] was much smaller than that of the previously known [EMI][Tf₂N] salt, and almost no change was observed even after 500 charge/discharge cycles. This suggests that the interface between the positive electrode and electrolyte solution was stably constructed.

The analysis was performed for the [EMI][Tf₃C] (Comparative Example 2) as well; however, an operation was unable to be performed from the first time due to too high internal resistance. In comparison with the results of the Tf₂N system, it is clear that the only replacement of the CF₃SO₂ group of Tf₃C with FSO₂ group reduced the viscosity and the interfacial charge-transfer resistance, which, as a result, reduced the internal resistance. This not only greatly improved the performance but also greatly improved the stability of the electrolyte solution at the positive-electrode interface.

### (4) Thermogravimetric Change

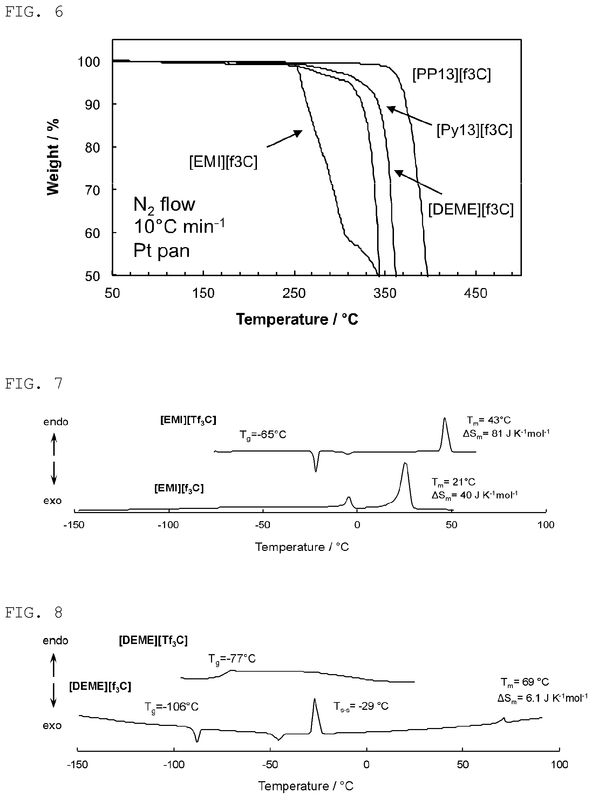

The thermogravimetric change of the [EMI][f₃C], [DEME][f₃C], [Py₁₃][f₃C], and [PP₁₃][f₃C] was observed in a nitrogen atmosphere. Fig. 6 shows the results. The results clarified that all of the salts had thermal resistance (about 250°C).

### (5) DSC

Fig. 7 shows the DSC measurement results of the [EMI][f₃C] (Example 1) and the [EMI][Tf₃C] (Comparative Example 2: [Tf₃C] = [(CF₃SO₂)₃C]) (at the time of temperature elevation; temperature elevation rate: 10°C per minute).

The results of Fig. 7 clarify that the [f₃C] anion of the present invention, when forming an ionic liquid with a typical EMI cation, gives a salt having a lower melting point.

Further, Fig. 8 shows the DSC measurement results of the [DEME] [f₃C] (Reference, Example 1) and the [DEME] [Tf₃C] (Comparative Example 2: [Tf₃C] = [(CF₃SO₂)₃C]) (at the time of temperature elevation; temperature elevation rate: 10°C per minute).

The results of Figs. 7 and 8 clarify that the [f₃C] anion of the present invention gives a salt having a melting point much higher than room temperature with the DEME cation, which most easily forms an ionic liquid next to EMI. However, its melting entropy is as significantly low as 6.1 J K⁻¹mol⁻¹, which indicates that this salt is a plastic crystal that is expected to be used as a unique solid electrolyte. Further, the [DEME][f₃C] had a much lower glass transition temperature, and presumably achieves very high ion mobility in the plastic crystal. Therefore, it is clear that the [f₃C] anion of the present invention is an anion that easily forms a plastic crystal.

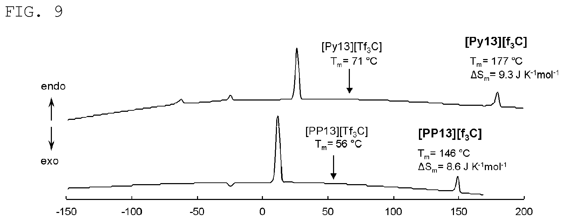

Further, Fig. 9 shows the DSC measurement results of the [Py₁₃][f₃C] (reference) and [PP₁₃][f₃C] (Example 1), and the [Py₁₃][Tf₃C] and [PP₁₃][Tf₃C] (Comparative Example 1: [Tf₃C] = [(CF₃SO₂)₃C]) (at the time of temperature elevation; temperature elevation rate: 10°C per minute).

With Py₁₃ and PP₁₃ cations as well, the f₃C salts had a melting point that is about 100°C higher than that of the Tf₃C salts, and a melting entropy that is greatly lower than the definition of the plastic crystal of Timmermans (ΔSₘ < 20 J K⁻¹ mol⁻¹, J. Timmermans, J. Phys. Chem. Solids, 18 (1), (1961), and references therein). This indicates that both of these f₃C salts were plastic crystals. For use as an electrolyte, plastic crystals that have a higher melting point, which is an upper-limit temperature that represents a plastic crystal phase, may be used in a wider temperature range as a solid electrolyte. The salt including f₃C anion exhibits a broad endotherm (equivalent to the temperature at which the salt becomes a plastic crystal) at around room temperature. This indicates that the f₃C anion is an excellent anion that provides a plastic crystal phase within a wide temperature range, i.e., room temperature or higher, which is important for practical use.

## Claims

1. A lithium secondary battery comprising
a Li metal negative electrode as a negative electrode and
a non-aqueous electrolyte for lithium secondary batteries, the electrolyte comprising an anion and a cation, the anion comprising [C(SO₂F)₃]⁻, and the cation comprising 1-ethyl-3-methylimidazolium ([EMI]⁺).

2. A lithium secondary battery according to Claim 1, wherein the non-aqueous electrolyte for lithium secondary batteries further comprises Li[C(SO₂F)₃].

3. A plastic crystal comprising an anion and a cation, the anion comprising [C(SO₂F)₃]⁻, and the cation comprising at least one member selected from the group consisting of N-methyl-N-propylpiperidinium ([PP₁₃]⁺), tetramethylammonium ([N_{1111]}⁺), tetraethylammonium ([N₂₂₂₂]⁺), and N-methyl-N-ethylpyrrolidinium ([Py₁₂]⁺).

4. A non-aqueous electrolyte for lithium secondary batteries, the electrolyte comprising an anion and a cation, the anion comprising [C(SO₂F)₃]⁻, and the cation comprising at least one member selected from the group consisting of N-methyl-N-propylpiperidinium ([PP₁₃]⁺), tetramethylammonium ([N₁₁₁₁]⁺), tetraethylammonium ([N₂₂₂₂]⁺), and N-methyl-N-ethylpyrrolidinium ([Py₁₂]⁺).

5. The non-aqueous electrolyte for lithium secondary batteries according to claim 4, further comprising Li[C(SO₂F)_{3]}.

6. A lithium secondary battery comprising the non-aqueous electrolyte of any one of claims 4 and 5.

7. The lithium secondary battery according to claim 6, comprising a Li metal negative electrode as a negative electrode.

## Patentansprüche

1. Lithiumsekundärbatterie, umfassend
eine negative Li-Metallelektrode als negative Elektrode und
einen nichtwässrigen Elektrolyten für Lithiumsekundärbatterien, wobei der Elektrolyt ein Anion und ein Kation umfasst, das Anion [C(SO₂F)₃]⁻ umfasst und das Kation 1-Ethyl-3-methylimidazolium ([EMI]⁺) umfasst.

2. Lithiumsekundärbatterie nach Anspruch 1, wobei der nichtwässrige Elektrolyt für Lithiumsekundärbatterien ferner Li[C(SO₂F)_{3]} umfasst.

3. Plastischer Kristall, der ein Anion und ein Kation umfasst, wobei das Anion [C(SO₂F)₃]⁻ umfasst und das Kation mindestens ein Mitglied umfasst ausgewählt aus der Gruppe bestehend aus N-Methyl-N-propylpiperidinium ([PP₁₃]⁺), Tetramethylammonium ([N₁₁₁₁⁺), Tetraethylammonium ([N₂₂₂₂]⁺) und N-Methyl-N-ethylpyrrolidinium ([Py₁₂]⁺.

4. Nichtwässriger Elektrolyt für Lithiumsekundärbatterien, wobei der Elektrolyt ein Anion und ein Kation umfasst, das Anion [C(SO₂F)₃]⁻ umfasst und das Kation mindestens ein Mitglied umfasst ausgewählt aus der Gruppe bestehend aus N-Methyl-N-propylpiperidinium ([PP₁₃]⁺), Tetramethylammonium ([N1111]⁺), Tetraethylammonium ([N₂₂₂₂]⁺) und N-Methyl-N-ethylpyrrolidinium ([Py₁₂]⁺).

5. Nichtwässriger Elektrolyt für Lithiumsekundärbatterien nach Anspruch 4, der ferner Li[C(SO₂F)₃] umfasst.

6. Lithiumsekundärbatterie, die den nichtwässrigen Elektrolyten gemäß einem der Ansprüche 4 und 5 umfasst.

7. Lithiumsekundärbatterie nach Anspruch 6, die eine negative Li-Metallelektrode als negative Elektrode umfasst.

## Revendications

1. Batterie secondaire au lithium comprenant
une électrode négative en Li métallique en tant qu'électrode négative et
un électrolyte non aqueux pour batteries secondaires au lithium, l'électrolyte comprenant un anion et un cation, l'anion comprenant [C(SO₂F)₃]⁻, et le cation comprenant le 1-éthyl-3-méthylimidazolium ([EMI]⁺).

2. Batterie secondaire au lithium selon la revendication 1, dans laquelle l'électrolyte non aqueux pour batteries secondaires au lithium comprend en outre Li[C(SO₂F)₃].

3. Cristal plastique comprenant un anion et un cation, l'anion comprenant [C(SO₂F)₃]⁻, et le cation comprenant au moins un élément sélectionné dans le groupe consistant en le N-méthyl-N-propylpipéridinium ([PP₁₃]⁺), le tétraméthylammonium ([N₁₁₁₁]⁺), le tétraéthylammonium ([N_{2222]}⁺), et le N-méthyl-N-éthylpyrrolidinium ([Py₁₂]⁺).

4. Électrolyte non aqueux pour batteries secondaires au lithium, l'électrolyte comprenant un anion et un cation, l'anion comprenant [C(SO₂F)₃]⁻, et le cation comprenant au moins un élément sélectionné dans le groupe consistant en le N-méthyl-N-propylpipéridinium ([PP₁₃]⁺), le tétraméthylammonium ([N₁₁₁₁]⁺), le tétraéthylammonium ([N₂₂₂₂]⁺), et le N-méthyl-N-éthylpyrrolidinium ([Py₁₂]⁺).

5. Électrolyte non aqueux pour batteries secondaires au lithium selon la revendication 4, comprenant en outre Li[C(SO₂F)₃].

6. Batterie secondaire au lithium comprenant l'électrolyte non aqueux selon l'une quelconque des revendications 4 et 5.

7. Batterie secondaire au lithium selon la revendication 6, comprenant une électrode négative en Li métallique en tant qu'électrode négative.
